# EUROPEAN PATENT APPLICATION

(11) **EP 4 026 551 A1**
(43) Date of publication of application: **13.07.2022**
(21) Application number: 20861449.5
(22) Date of filing: 04.09.2020
(51) Int. Cl.: A61K 35/545, A61K 35/28, A61P 9/04, C12N 5/0775

(54) **THERAPEUTIC AGENT FOR MYOCARDITIS**

(30) Priority: 05.09.2019 JP 2019162264
(71) Applicant: Tohoku University, Sendai-shi, Miyagi 980-8577 (JP); Life Science Institute, Inc., Tokyo 100-8251 (JP)
(72) Inventor: DEZAWA, Mari, Sendai-shi, Miyagi 980-8577 (JP); SAIKI, Yoshikatsu, Sendai-shi, Miyagi 980-8577 (JP); TAKAYA, Hiroki, Sendai-shi, Miyagi 980-8577 (JP)
(74) Representative: Gill Jennings & Every LLP
(86) International application number: PCT/JP2020/033577
(87) International publication number: WO 2021/045190

(57) **Abstract**

Provided is a cell product for treating myocarditis, comprising a SSEA-3-positive pluripotent stem cell derived from a mesenchymal tissue in a living body or a SSEA-3-positive pluripotent stem cell derived from a cultured mesenchymal cell.

## Description

### TECHNICAL FIELD

The present invention relates to a cell product for regenerative therapy. More specifically, the present invention relates to a cell product comprising a pluripotent stem cell that is effective in therapy of myocarditis.

### BACKGROUND ART

Myocarditis is an inflammatory disease primarily involving the myocardium. When inflammation extends to pericardium, it is called pericardial myocarditis. There are a variety of types of myocarditis, including acute myocarditis, chronic myocarditis, fulminant myocarditis, dilated cardiomyopathy-like type, and the like, and the onset period can range from a few hours to a week or two, or even longer, and the prognosis can vary from complete normalization to death.

In most cases, the onset of inflammation of the myocardium is triggered by viral infections. Other causes of inflammation are said to include bacterial and other infections, drugs, chemical substances, allergies, autoimmune diseases, collagen diseases, Kawasaki disease, sarcoidosis, radiation, heatstroke, and AIDS. In some cases, myocarditis develops without an obvious cause being specified.

The frequency of myocarditis is not known, but studies have shown that the frequency is 115 per 100,000 population.

Since myocarditis is often triggered by a viral infection, a cold symptom (chill, fever, headache, myalgia, general malaise, or the like) or a gastrointestinal symptom such as anorexia, nausea, vomiting, or diarrhea appears first as a sign. As the disease progresses, (1) a sign of heart failure, (2) chest pain due to pericardial stimulation, and (3) a cardiac symptom associated with heart block or arrhythmia may appear, and when fatal arrhythmia develops, lightheadedness or fainting may occur, leading to sudden death.

In the case of eosinophilic myocarditis, giant cell myocarditis, or the like, a steroid or an immunosuppressive agent is used to treat myocarditis, but in many cases, myocarditis is triggered by a viral infection, which often prevents radical therapy.

For this reason, short-term high-dose steroid therapy, high-dose immunoglobulin therapy, and plasma exchange therapy are being considered to relieve myocardial function suppression by inflammatory substances.

In the course of myocarditis, when dealing with heart failure or fatal arrhythmias is needed, a diuretic or a pressure booster is used, artificial respiration is managed, an assisted circulation device is used, or pacemaker therapy is used. For assisted circulation which helps heart function, intra-aortic balloon pumping, percutaneous cardiopulmonary assist device, artificial heart, or the like is also used.

As described above, symptomatic treatment of myocarditis is being used and there is no radical cure for myocarditis, and therefore, there is an urgent need to provide an effective medicine for direct treatment of myocarditis.

On the other hand, recent progress in regenerative therapy research has led to exploration of treatments for myocarditis such as bone marrow stem cell transplantation.

For example, Non-patent Document 1 discloses that bone marrow-derived mesenchymal stem cells reduce myocardial damage and dysfunction in a rat model of acute myocarditis.

However, the efficacy of such treatment has not yet been fully established, and the clinical benefits are unknown.

It has been found in researches by the present inventor Dezawa et al. that pluripotent stem cells, which are present in mesenchymal cell fractions, can be obtained without gene introduction or induction by cytokines or the like, and express SSEA-3 (Stage-Specific Embryonic Antigen-3) as a surface antigen (Multilineage-differentiating Stress Enduring cells; Muse cell), can be responsible for the pluripotency possessed by the mesenchymal cell fractions, and applied to disease treatment aimed at tissue regeneration (e.g., Patent Document 1; Non-patent Documents 2 to 4). It is known that Muse cells can be obtained from bone marrow aspirates, adipose tissue (Non-patent Document 5), dermal connective tissue of skin, or the like, and are widely present in tissues or connective tissues of organs.

However, effects of Muse cells on myocarditis are not known.

### PRIOR ART DOCUMENTS

### PATENT DOCUMENTS

Patent Document1: Japanese Patent 5185443

### NON-PATENT DOCUMENTS

Non-patent Document 1: Ohnishi S et al. J Mol Cell Cardio. 2007 Jan; 42(1): 88-97.
Non-patent Document 2: Kuroda Y et al. Proc Natl Acad Sci USA 2010; 107: 8639-8643.
Non-patent Document 3: Wakao S et al. Proc Natl Acad Sci USA 2011; 108: 9875-9880.
Non-patent Document 4: Kuroda Y et al. Nat Protc 2013; 8: 1391-1415.
Non-patent Document 5: Ogura, F., et al., Stem Cells Dev, 2013 Nov; 23; 7

### SUMMARY OF THE INVENTION

### PROBLEMS TO BE SOLVED BY THE INVENTION

An object of the present invention is to provide a cell product for treating myocarditis.

### MEANS FOR SOLVING THE PROBLEMS

The present inventors, as a result of intensive study to solve the above-described problem, found that Muse cells can accumulate in an impaired myocardial tissue caused by myocarditis, differentiate into cardiomyocytes in the impaired myocardial tissue, and bring about repair of myocardial tissue, reduction of the impaired myocardial tissue, and improvement or recovery of cardiac function.

Accordingly, the present invention provides the following items [1] to [11].
[1] A cell product for treating myocarditis, comprising a SSEA-3-positive pluripotent stem cell derived from a mesenchymal tissue in a living body or a SSEA-3-positive pluripotent stem cell derived from a cultured mesenchymal cell.
[2] The cell product of item [1], wherein the pluripotent stem cell contains a concentrated cell fraction.
[3] The cell product of item [1] or [2], wherein the pluripotent stem cell is CD105-positive.
[4] The cell product of any one of items [1] to [3], wherein the pluripotent stem cell is CD117-negative and CD146-negative.
[5] The cell product of any one of items [1] to [4], wherein the pluripotent stem cell is CD117-negative, CD146-negative, NG2-negative, CD34-negative, vWF-negative, and CD271-negative.
[6] The cell product of any one of items [1] to [5], wherein the pluripotent stem cell is CD34-negative, CD117-negative, CD146-negative, CD271-negative, NG2-negative, vWF-negative, SoxlO-negative, Snail-negative, Slug-negative, Tyrp1-negative, and Dct-negative.
[7] The cell product of any one of items [1] to [6], wherein said pluripotent stem cell is one having all of the following characteristics:
   (i) having low or no telomerase activity;
   (ii) capable of differentiating into any of tridermic cells;
   (iii) showing no neoplastic proliferation; and
   (iv) having self-renewal capacities.
[8] The cell product of item [7], wherein said pluripotent stem cell is one having all of the following characteristics:
   (i) SSEA-3-positive;
   (ii) CD105-positive;
   (iii) having low or no telomerase activity;
   (iv) capable of differentiating into any of tridermic cells;
   (v) showing no neoplastic proliferation; and
   (vi) having self-renewal capacities.
[9] The cell product of any one of items [1] to [8], wherein the myocarditis is one or two or more selected from the group consisting of lymphocytic myocarditis, giant cell myocarditis, eosinophilic myocarditis, and granulomatous myocarditis.
[10] Use of a SSEA-3-positive pluripotent stem cell derived from a mesenchymal tissue in a living body or a SSEA-3-positive pluripotent stem cell derived from a cultured mesenchymal cell, in manufacture of a cell product for treating or preventing myocarditis.
[11] A method of treating myocarditis, comprising administering an effective amount of a cell product comprising a SSEA-3-positive pluripotent stem cell derived from a mesenchymal tissue in a living body or a SSEA-3-positive pluripotent stem cell derived from a cultured mesenchymal cell to a patient with myocarditis in need of treatment or prevention.

### EFFECTS OF THE INVENTION

According to the present invention, a cell product for treating myocarditis, containing Muse cells is provided.

Since Muse cells can efficiently migrate and engraft to a site of myocardial tissue damage due to myocarditis, and are considered to spontaneously differentiate at the engraftment site, they do not require induction of differentiation into cells to be treated prior to transplantation. Muse cells are non-tumorigenic and excellent in safety. Furthermore, since Muse cells do not induce any immune rejection, treatment with allogenic preparations produced from donors is also possible. Therefore, Muse cells having the excellent characteristics as described above can provide readily feasible means for treating myocarditis.

### BRIEF DESCRIPTION OF THE DRAWINGS

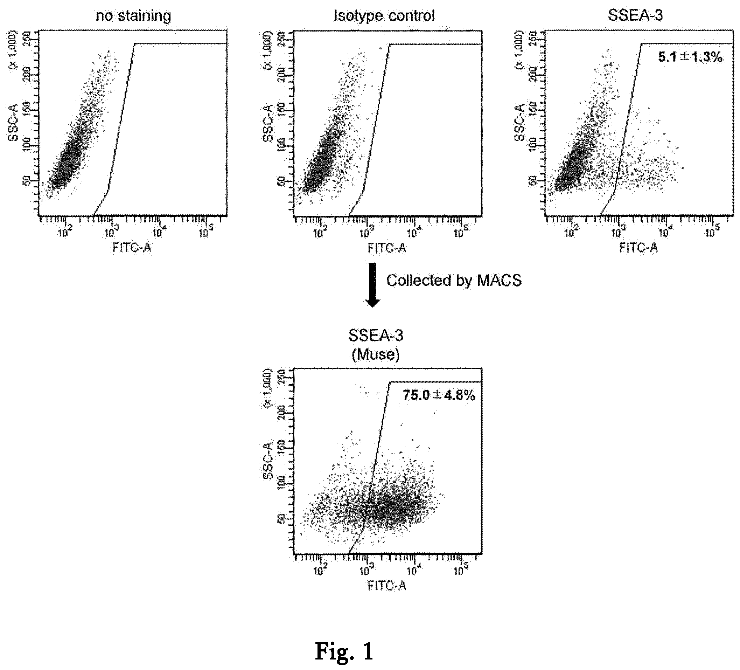

FIG. 1 represents the results of cell analysis by FACS. The upper row from left to right illustrates the results of unstained cell population, cell population using isotype control, and cell population using SSEA-3 antibody. The lower row illustrates the results of an SSEA-3-positive cell population enriched by MACS.

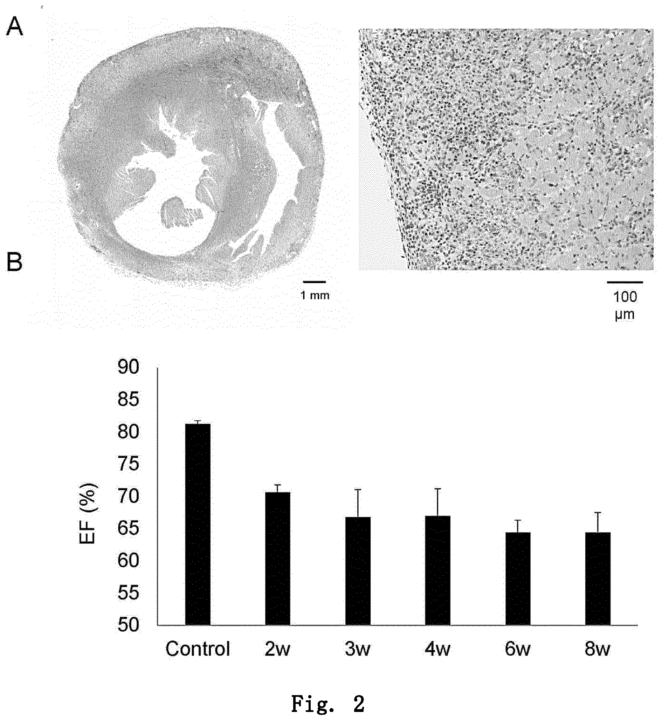

FIG. 2 illustrates the results of cardiac function measurements in myocarditis rats. A in FIG. 2 is the light microscopic images (substitute photograph for drawing) of HE staining of the cardiac tissue (paraffin section) from a rat with myocarditis. The right image is a magnified image of the left image. B in FIG. 2 illustrates the ejection fraction (EF) obtained from MRI imaging data.

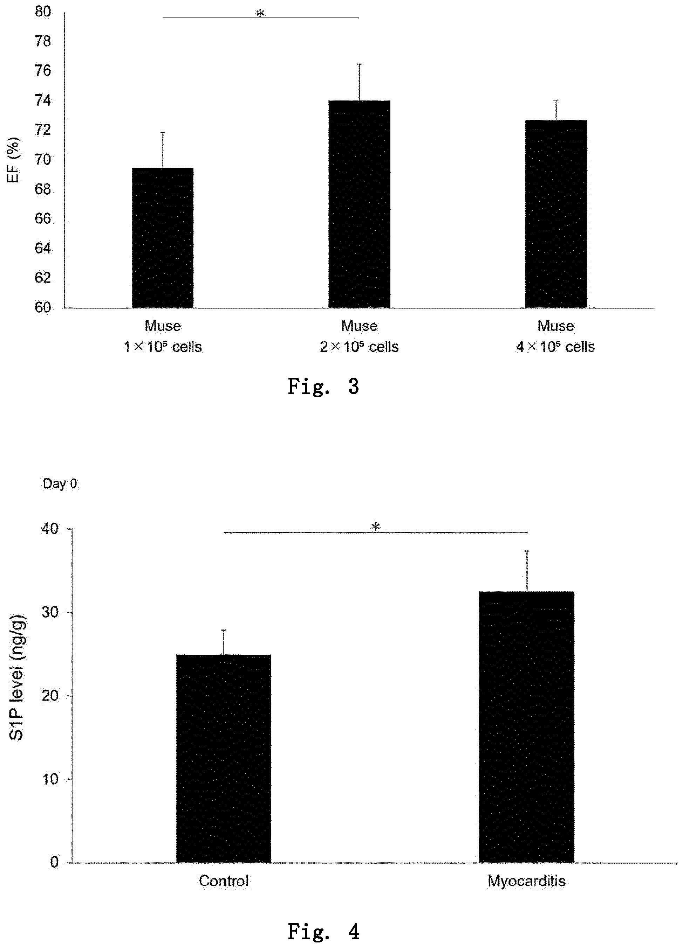

FIG. 3 illustrates the results of cardiac function measurements (EF) of the cell-transplanted rats with different cell numbers.
FIG. 4 illustrates the results of sphingosine-1-phosphate (SIP) concentration measurement in the cell-transplanted rats at 0 day after transplantation.

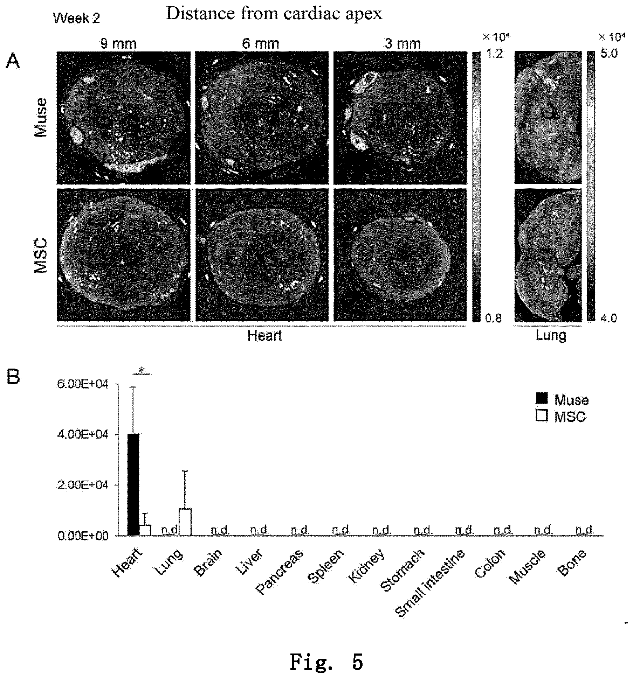

FIG. 5 illustrates the in vivo distribution of the cells administered in the cell-transplanted rats. A in FIG. 5 is an analytical image by an in vivo imaging system (substitute photograph for drawing). B in FIG. 5 illustrates the light intensity of each organ obtained by the in vivo imaging system.

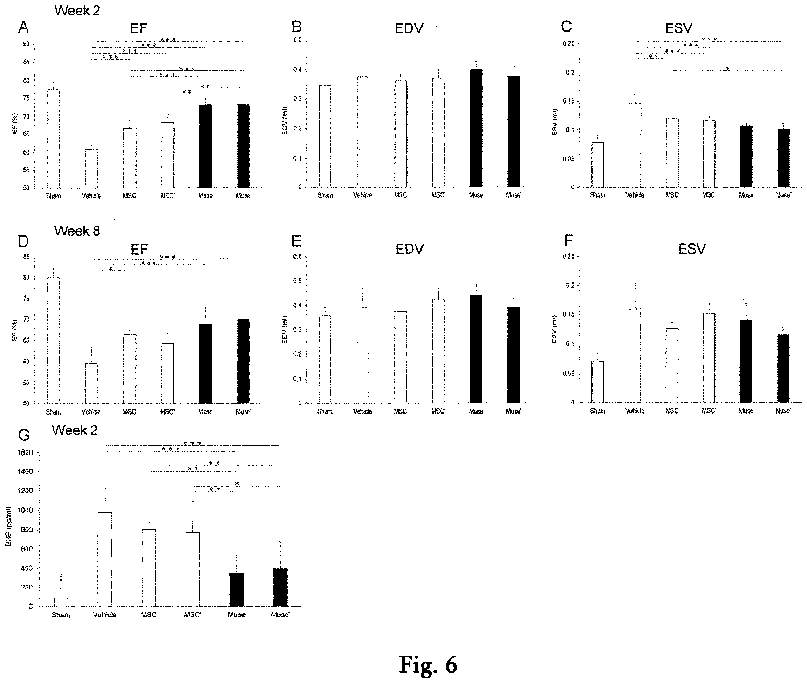

FIG. 6 illustrates the results of cardiac function measurements in the cell-transplanted rats. A-F in FIG. 6 illustrate the measurement results of cardiac function (left ventricular end-diastolic volume (EDV), left ventricular end-systolic volume (ESV), and ejection fraction (EF)) at two and eight weeks after transplantation. G in FIG. 6 illustrates the measurement results of cardiac function (brain natriuretic peptide (BNP)) at two weeks after transplantation.

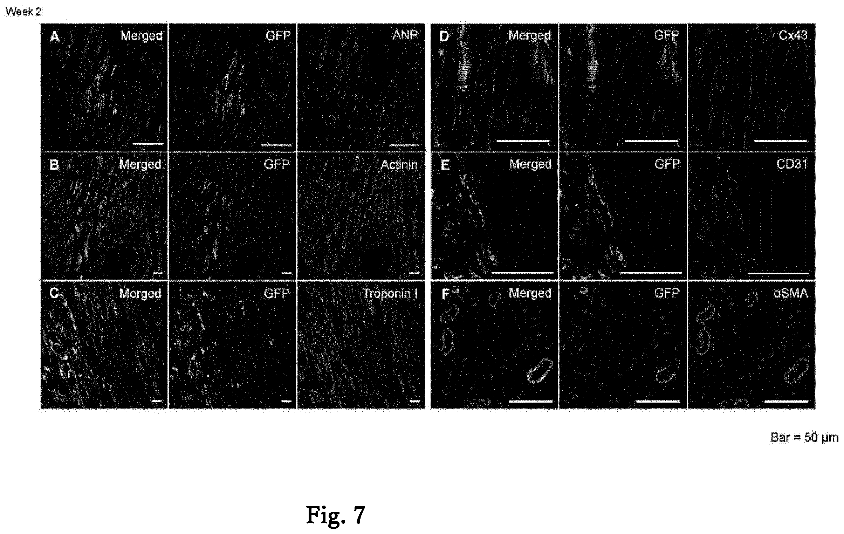

FIG. 7 illustrates the results (substitute photograph for drawing) of histological evaluation of the cell-transplanted rats. A-F in FIG. 7 illustrate the detection results of immature myocardial marker ANP, mature myocardial markers sarcomeric α-actinin, troponin-I, and connexin 43, and vascular component cells CD31 and αSMA, respectively. In A-F, the left panels illustrate merged images, the center panels illustrate GFP, and the right panels illustrate the detection results of respective myocardial markers.

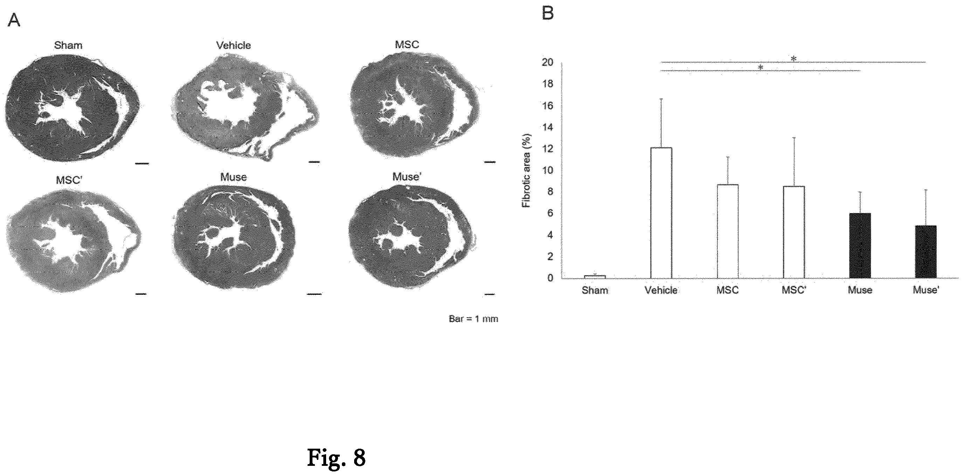

FIG. 8 illustrates the results of histological evaluation of the cell-transplanted rats. A in FIG. 8 is an optical microscope image of Masson-Trichrome staining of paraffin sections of heart tissue of each cell-treated group eight weeks after transplantation (substitute photograph for drawing). B in FIG. 8 illustrates the cardiac fibrotic area/cardiac sectional area calculated from the optical microscope images.

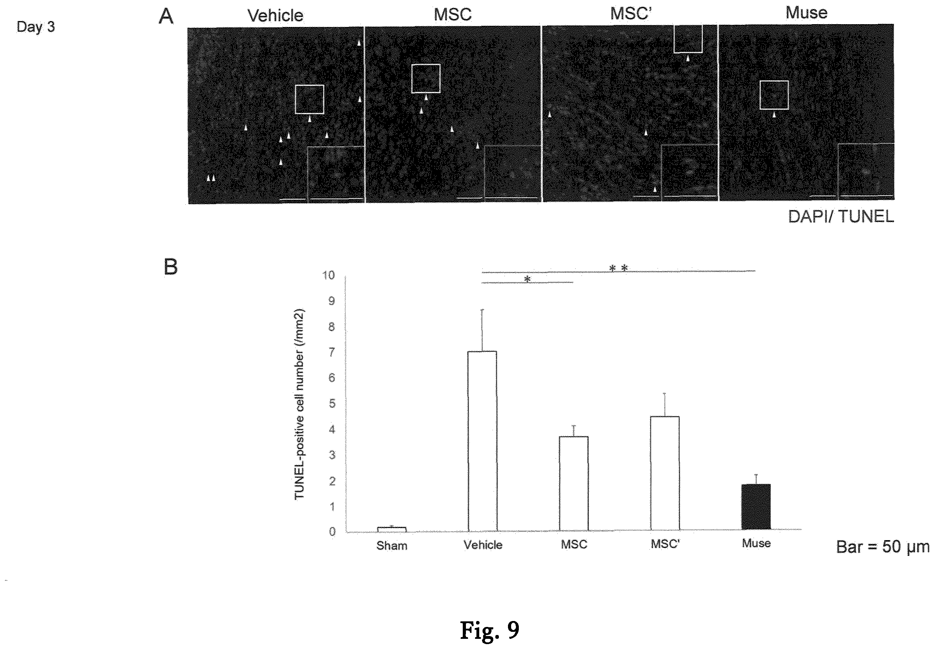

FIG. 9 illustrates the measurement results of the number of apoptotic cells in the tissues of the cell-transplanted rats. A in FIG. 9 is an optical microscope image of TdT-mediated dUTP nick end labeling (TUNEL) staining of frozen sections of heart tissue of each cell-treated group three days after transplantation (substitute photograph for drawing). B in FIG. 9 illustrates the number of TUNEL-positive cells.

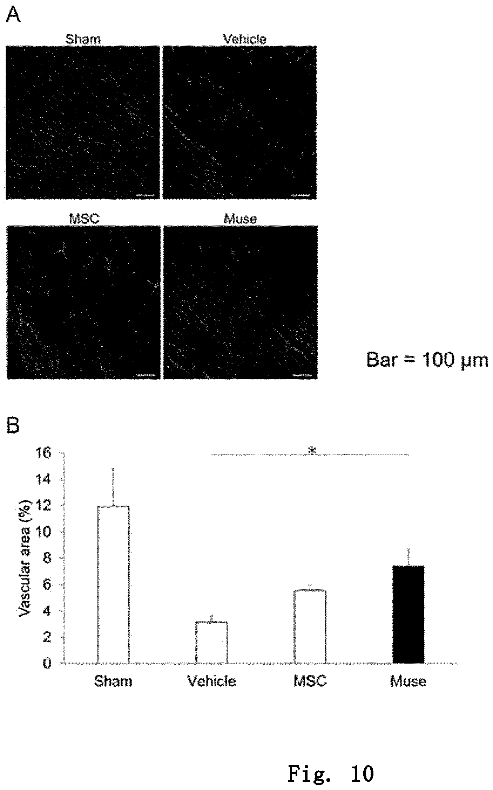

FIG. 10 illustrates the evaluation results of angiogenesis by cell transplantation. A in FIG. 10 is the tomographic images of groups of blood vessels in clarified cardiac tissues taken by multiphoton laser microscopy. B in FIG. 10 illustrates the ratios of the vessel volumes in the heart tissues.

### DETAILED DESCRIPTION OF THE INVENTION

The present invention will now be described.

<1> A cell product comprising a SSEA-3-positive pluripotent stem cell (Muse cell) derived from a mesenchymal tissue in a living body or a SSEA-3-positive pluripotent stem cell derived from a cultured mesenchymal cell.

The present invention relates to a cell product (hereinafter, referred to as "cell product of the present invention") for treating myocarditis, comprising a Muse cell.

In the present invention, "treatment of myocarditis" includes treatment and improvement of myocarditis such as reparation of impaired myocardial tissue caused by myocarditis, reduction of impaired myocardial tissue, and improvement or restoration of cardiac function, as well as healing, alleviation, and prevention of recurrence of symptoms of myocarditis.

### 1. Applicable diseases

Cell products comprising the Muse cell of the present invention are used for treating myocarditis.

Myocarditis is classified into lymphocytic myocarditis, giant cell myocarditis, eosinophilic myocarditis, and granulomatous myocarditis based on histological features. In terms of etiology, lymphocytic myocarditis is often caused by viral infections, while giant cell myocarditis, eosinophilic myocarditis, and granulomatous myocarditis are often regarded as complications of cardiotoxic substances, drug allergies, autoimmunity, and systemic diseases.

According to the mode of onset, myocarditis can be classified into acute myocarditis and chronic myocarditis. Among acute myocarditis, those that lead to cardiopulmonary crisis in the early stage of onset are called fulminant myocarditis. Chronic myocarditis refers to myocarditis that persists for a relatively long period of time, for example, more than a few months, often resulting in heart failure and arrhythmia, and a condition similar to dilated cardiomyopathy. Chronic cardiomyopathy can be classified into those with subclinical onset and a chronic course (subclinical) and those with persistent acute myocarditis (persistent).

Another inflammatory disease of the heart is pericarditis, and sometimes pericarditis is associated with myocarditis. In such a case, the disease is called pericarditis/myocarditis.

Furthermore, acute myocarditis may be the cause of dilated cardiomyopathy.

In the present invention, "myocarditis" includes all of the above. Although not limited, examples of myocarditis targeted by the cell product of the present invention preferably include autoimmune myocarditis, and more preferably include giant cell myocarditis.

### 2. Cell product

### (1) SSEA-3-positive pluripotent stem cell (Muse cell) derived from a mesenchymal tissue in a living body or the cell derived from a cultured mesenchymal cell

The pluripotent stem cell used in the cell product of the present invention is a cell that was found in human living body and named "Muse (Multilineage-differentiating Stress Enduring) cell" by the present inventor Dezawa. It is known that Muse cells can be obtained from, for example, bone marrow aspirates, adipose tissues (Ogura, F., et al., Stem Cells Dev., Nov 20, 2013 (Epub) (published on Jan 17, 2014)) and dermal connective tissues of skin, and are also broadly present in tissues and connective tissues in organs. This cell also has both characteristics of pluripotent stem cell and mesenchymal stem cell and is identified as, for example, a cell positive for "SSEA-3 (Stage-specific embryonic antigen-3)," a cell surface marker, preferably as a double-positive cell that is positive for SSEA-3 and CD-105. Therefore, Muse cells or a cell population containing Muse cells can be separated from living tissues using, for example, expression of SSEA-3 only or a combination of SSEA-3 and CD-105 as an index. Taking advantage of the high resistance of Muse cells to various external stresses, Muse cells can be selectively enriched by culturing the cells under various external stress conditions, such as under protease treatment, under hypoxic conditions, under low phosphate conditions, in a low serum concentration, under undernutrition conditions, under heat shock exposure, in the presence of toxic substances, in the presence of reactive oxygen species, under mechanical stimulation, and under pressure treatment. Methods for separation and identification of, characteristics and concentration of Muse cells have been disclosed in WO 2011/007900 in detail.

As used herein, pluripotent stem cells prepared from mesenchymal tissues in a living body or from cultured mesenchymal tissues using SSEA-3 as an index (Muse cells), or a cell population comprising Muse cells, as a cell product for treating peripheral arterial diseases, may be simply referred to as "SSEA-3-positive cells."

Muse cells or a cell population comprising Muse cells can be prepared from living tissues (e.g., mesenchymal tissues) using cell surface markers, SSEA-3, or SSEA-3 and CD-105, as an index(es). As used herein, the term "living" body means mammal living body.

In the present invention, living bodies exclude fertilized egg and embryos in developmental stages before blastula stage, but include embryos in developmental stages of blastula stage or later, including fetus and blastula. Examples of the mammal include, but not limited to, primates such as human and monkey; rodents such as mouse, rat, rabbit, and guinea pig; and cat, dog, sheep, pig, cattle, horse, donkey, goat, and ferret.

Muse cells to be used in the cell product of the present invention are directly separated from living tissues using markers, and thus are clearly distinguished from embryonic stem cells (ES cells) and induced pluripotent stem (iPS) cells.

The term "mesenchymal tissue" refers to tissues containing mesenchymal cells such as bone, synovial membrane, fat, blood, bone marrow, skeletal muscle, dermis, ligament, tendon, dental pulp, umbilical cord, cord blood, and amnion, as well as tissues present in various organs. For example, Muse cells can be obtained from bone marrow, skin, adipose tissues, blood, dental pulp, umbilical cord, cord blood, or amnion. For example, and preferably, a mesenchymal tissue in a living body is collected, and then Muse cells are prepared from the tissue and used. Alternatively, using the preparation method described above, Muse cells may be prepared from cultured mesenchymal cells such as fibroblasts or bone marrow mesenchymal stem cells.

The cell population comprising Muse cells to be used in the cell product of the present invention can also be prepared by a method comprising stimulating a mesenchymal tissue in a living body or cultured mesenchymal cells with an external stress to selectively increase cells that are resistant to the external stress, and collecting the cells with an increased abundance ratio.

The external stress may be any one of or a combination of the following: protease treatment, culturing under low oxygen concentration, culturing under low phosphate conditions, culturing under low serum concentration, culturing undernutrition conditions, culturing under heat shock exposure, culturing at low temperatures, freezing treatment, culturing in the presence of toxic substances, culturing in the presence of reactive oxygen species, culturing under mechanical stimulation, culturing under shaking, culturing under pressure treatment or physical shocks.

The protease treatment is preferably carried out for 0.5 to 36 hours in total to exert an external stress. The concentration of the protease is preferably used when cells adhered to a culture vessel are peeled off, when cell aggregates are separated into single cells, or when single cells are collected from a tissue.

Preferably, the protease is a serine protease, an aspartic protease, a cysteine protease, a metalloprotease, a glutamic protease, or an N-terminal threonine protease. More preferably, the protease is trypsin, collagenase, or Dispase.

Muse cells to be used in the cell product of the present invention may be autologous or allogeneic to a recipient who will receive the cells.

As described above, Muse cells or a cell population comprising Muse cells can be prepared from living tissues, for example, by using SSEA-3 positivity or SSEA-3 and CD-105 double positivity as an index. Human adult skin is known to comprise various types of stem cells and progenitor cells. However, Muse cells are different from these cells. These stem cells and progenitor cells include skin-derived progenitor cells (SKP), neural crest stem cells (NCSC), melanoblasts (MB), pericytes (PC), endothelial progenitor cells (EP), and adipose-derived stem cells (ADSC). Muse cells can be prepared using "non-expression" of markers unique to these cells as an index. More specifically, Muse cells can be separated using as an index non-expression of at least one, e.g., 2, 3, 4, 5, 6, 7, 8, 9, 10, or 11, of 11 markers selected from the group consisting of CD34 (a marker for EP and ADSC), CD117 (c-kit) (a marker for MB), CD146 (a marker for PC and ADSC), CD271 (NGFR) (a marker for NCSC), NG2 (a marker for PC), vWF factor (von Willebrand factor) (a marker for EP), Sox10 (a marker for NCSC), Snail (a marker for SKP), Slug (a marker for SKP), Tyrp1 (a marker for MB), and Dct (a marker for MB). Muse cells can be prepared by using as an index non-expression of, for example, but not limited to, CD117 and CD146; CD117, CD146, NG2, CD34, vWF, and CD271; or the above-described 11 markers.

Muse cells having the above-described characteristics and used in the cell product of the present invention may also have at least one selected from the group consisting of the following characteristics:
(i) having low or no telomerase activity;
(ii) capable of differentiating into any of tridermic cells;
(iii) showing no neoplastic proliferation; and
(iv) having self-renewal capacities.

Preferably, Muse cells to be used in the cell product of the present invention has all of the characteristics described above.

With respect to (i) above, the phrase "having low or no telomerase activity" means that the telomerase activity is low or undetectable when detected using a known method for detecting telomerase activity such as a TRAPEZE XL telomerase detection kit (Millipore Corporation). Having "low" telomerase activity means, for example, having a telomerase activity comparable to somatic human fibroblast, or having 1/5 or less telomerase activity, preferably 1/10 or less telomerase activity, as compared with that of HeLa cell.

With respect to (ii) above, Muse cells are capable of being differentiated into tridermic cells (endodermal, mesodermal, and ectodermal cells) in vitro and in vivo, and can be differentiated into, for example, hepatocytes (including cells expressing markers of hepatoblast or hepatocyte), neurons, skeletal muscle cells, smooth muscle cells, osteocytes, or adipocytes by in vitro inductive culturing. Muse cells may also show the ability to be differentiated into tridermic cells when transplanted in testis in vivo. Further, Muse cells are capable of migrating and engrafting to injured organs (such as heart, skin, spinal cord, liver, and muscle) when transplanted into a living body via intravenous injection and being differentiated into cells depending on the tissues. The term "capable of differentiating into any of tridermic cells" refers to such capability of differentiation.

With respect to (iii) above, Muse cells are characterized in that they proliferate at a growth rate of about 1.3 days and proliferate from a single cell in suspension culture to form embryoid body-like cell aggregates, and then arrest their proliferation after about 14 days when the aggregates reach a certain size. When these embryoid body-like cell aggregates are transferred to adherent culture, the cells restart proliferation and cells proliferated from the cell aggregates expand at a growth rate of about 1.3 days. Further, Muse cells are characterized in that, when transplanted into testis, they do not become tumorigenic for at least half a year. The term "showing no neoplastic proliferation" refers to such non-neoplastic proliferation potential.

With respect to (iv) above, Muse cells have self-renewal (self-replication) capacities. The term "having self-renewal capacities," as used herein, means that the followings can be observed: differentiation into tridermic cells from cells contained in first embryoid body-like cell aggregates obtained by culturing single Muse cells in a suspension culture; as well as formation of next-generation second embryoid body-like cell aggregates by again culturing single cells in the first embryoid body-like cell aggregates in a suspension culture; and further differentiation into tridermic cells and formation of third embryoid body-like cell aggregates in a suspension culture from the second embryoid body-like cell aggregates. Self renewal may be repeated for one or more cycles.

### (2) Preparation and use of cell product comprising SSEA-3-positive pluripotent stem cell (Muse cell) derived from a mesenchymal tissue in a living body or the cell derived from a cultured mesenchymal cell

The cell product comprising Muse cells of the present invention can be obtained by, but not limited to, suspending Muse cells or a cell population comprising Muse cells defined in (1) above in a physiological saline or a suitable buffer solution (e.g., a phosphate buffered saline). In this case, when only small numbers of Muse cells are separated from an autologous or allogeneic tissue, these cells may be cultured before cell transplantation until the predetermined number of cells is attained. As previously reported (WO2011/007900), since Muse cells are non-tumorigenic, they are less likely to be tumorigenic and thus are safe, even if cells collected from a living tissue are contained in undifferentiated states. The collected Muse cells can be cultured in any normal growth medium (e.g., alpha-minimum essential medium (a-MEM) supplemented with 10% calf serum). More specifically, with reference to the above-described WO2011/007900, Muse cells can be cultured and proliferated using an appropriately selected culture medium, additives (e.g., antibiotics, and serum) and the like, to prepare a solution containing Muse cells at a predetermined concentration.

When the cell product comprising Muse cells of the present invention is administered to a human subject, bone marrow aspirates are collected from a human ilium. Then, for example, bone marrow mesenchymal stem cells are cultured as adherent cells obtained from the bone marrow aspirate and proliferated until reaching the cell amount where a therapeutically effective amount of Muse cells can be obtained. Thereafter, Muse cells are separated using an antigenic marker SSEA-3 as an index to prepare a cell product containing autologous or allogeneic Muse cells. Alternatively, for example, bone marrow mesenchymal stem cells obtained from the bone marrow aspirates can be cultured under external stress conditions, so that Muse cells can be grown and enriched until they reach a therapeutically effective amount, thereby preparing a cell product comprising autologous or allogeneic Muse cells.

When Muse cells are used in a cell product, the cell product may also comprise dimethyl sulfoxide (DMSO), serum albumin and the like for protection of the cells and antibiotics and the like for prevention of contamination and proliferation of bacteria. The cell product may further comprise other pharmaceutically acceptable components (e.g., carriers, excipients, disintegrants, buffer agents, emulsifiers, suspending agents, soothing agents, stabilizers, preservatives, antiseptics, physiological saline). These agents and drugs can be added to the cell product at appropriate concentrations by the skilled person. Thus, Muse cells can also be used as a pharmaceutical composition comprising various additives (preferably as a regenerative medicine pharmaceutical composition).

The number of Muse cells contained in the cell product prepared above can be appropriately adjusted to achieve desired effects in treatment of myocarditis, in consideration of, for example, sex, age, and weight of the subject, the condition of the affected area, and the condition of the cells to be used. Individuals as the subject include, but are not limited to, mammals such as humans, as long as they have myocarditis, are suspected to have myocarditis, or at post-myocarditis. The cell product comprising Muse cells of the present invention may be administered once or a plularity of times at appropriate intervals (e.g., twice a day, once a day, twice a week, once a week, once every two weeks, once a month, once every two months, once every three months, or once every six months) until the desired therapeutic effect is obtained. Thus, the therapeutically effective amount is preferably, for example, 1 to 10 doses of 1×10³ to 1×10¹⁰ cells/individual/dose per year, depending on the state of the subject. The total amount administered to an individual is, but not limited to, 1×10³ to 1 ×10¹¹ cells, preferably 1×10⁴ to 1×10¹⁰ cells, more preferably 1×10⁵ to 1×10⁹ cells.

The dosage, number of times of administration, and the like of the cell product comprising Muse cells of the present invention can also be set based on an effect on a myocarditis model. The myocarditis model can be produced based on known methods, and examples thereof include, but not limited to, a rat model of autoimmune myocarditis (Kodama M, Matsumoto Y, Fujiwara M, Masani F, Izumi T, Shibata A. A novel experimental model of giant cell myocarditis induced in rats by immunization with cardiac myosin fraction. Clin Immunol Immunopathol. 1990; 57(2): 250-262.) and a Coxsackie B3 virus myocarditis mouse (Silver MA, Kowalczyk D. Coronary microvascular narrowing in acute murine coxsackie B3 myocarditis. Am Heart J. 1989; 118:173-174.), which are considered to be human giant cell myocarditis models.

Muse cells used in the cell product of the present invention are characterized by migrating to the impaired site and engrafting at the impaired site. Therefore, the site and method of administration of the cell product are not limited, and examples thereof include intravascular administration (intravenous, intra-arterial) and local administration.

The cell product comprising Muse cells of the invention can achieve repair and regeneration of the impaired site in a patient with myocarditis.

In one embodiment of the present invention, administration of the cell product of the present invention can reduce the size of impaired tissue due to myocarditis in a subject with myocarditis. Impaired tissue can be, but not limited to, tissue in which one or two or more of inflammatory cell infiltration, cardiomyocyte degeneration or interstitial edema, and fibrosis are observed. Herein, the term "impaired tissue size" is defined as the ratio (%) of impaired tissue to normal cardiac tissue. Impaired tissue size can be measured by normal examination, analysis, and measurement methods. When examining an effect of the cell product of the present invention on reducing the impaired tissue size, it is useful to use the rate of reduction relative to the impaired tissue size of a control (or (impaired tissue size of the control - impaired tissue size after cell transplantation)/impaired tissue size of the control × 100). According to the present invention, the impaired tissue size is preferably reduced by 100% with respect to a non-administered group of the cell product (control). The reduction is more preferably 10 to 90%, still more preferably 20 to 70%, and still more preferably 30 to 50%. In Examples below, when using rat cardiomyopathy models, in the control group, the impaired tissue size was 12.1 ± 4.5%, whereas in the Muse cell transplantation group, the impaired tissue size was 6.0 ± 2.0% or 4.8 ± 3.4%. These figures indicate that Muse cell transplantation was able to reduce the impaired tissue size by about 50 to 60%.

In one embodiment of the present invention, the cell product of the present invention can improve or restore the cardiac function after the onset of myocarditis to normal (or normal values). As used herein, the term "improvement" of cardiac function means improvement of cardiac function that has been impaired by myocarditis, and cardiac function is preferably improved to an extent that daily life is not affected. Restoring cardiac function to normal means that the cardiac function that has been reduced by myocarditis returns to the condition before myocarditis. In one aspect of the invention, the cell product of the present invention can be used for prevention and/or treatment of (chronic) heart failure after myocarditis.

Here, the index for evaluating cardiac function is not limited, and common examples thereof include end-diastolic volume (EDV), end-systolic volume (ESV), ejection fraction (EF), and brain natriuremic peptide (BNP). These indexes can be measured by common examination, analysis, and measurement methods. Improvement or recovery of cardiac function by the cell product of the present invention can be determined, for example, by using at least one of the above-described four indexes. For example, in Examples below, after 8 weeks of cell transplantation, in a Muse cell-transplant group, EF is 68.8±4.5% or 70.1±3.4%, and in the control group EF is 59.5±3.9%, and both measurements indicate that cardiac function is significantly improved in the cell transplant group compared to the control group.

Two weeks after cell transplantation, in the Muse cell-transplantation group, the plasma BNP level was 347.2±188.6 pg/ml or 395.2±283.3 pg/ml, and in the control group, the plasma BNP level was 980±241.1 pg/ml, and both measurements indicate that cardiac function was significantly improved in the cell-transplant group compared to the control group.

### EXAMPLES

The present invention will be described in more detail with reference to Examples below, but is not limited to the Examples.

### << Experimental Methods >>

### < Preparation of Myocarditis Model >

Eight-week-old male Lewis rats (LEW/SsNSlc, SLC Japan, Hamamatsu, Japan) were used. Under sedation by isoflurane inhalation anesthesia, autoimmune myocarditis was created by subcutaneous injection of 0.1 ml of Freund's complete adjuvant (Difco Laboratories, Sparks, MD, USA) containing swine myocardial myosin (10mg/ml; Sigma-Aldrich Japan, Tokyo, Japan) and the same amount of 11 mg/ml Myocobacterium tuberculosis into the sole of a foot, with reference to the prior document (Kodama M, Matsumoto Y, Fujiwara M, Masani F, Izumi T, Shibata A. A novel experimental model of giant cell myocarditis induced in rats by immunization with cardiac myosin fraction. Clin Immunol Immunopathol. 1990;57(2):250-262.).

### < Separation of Muse Cells >

Human bone marrow-derived mesenchymal stem cells (Mesenchymal Stem Cells: MSC) (Lonza Japan, Tokyo, Japan) were used. In accordance with the prior document (Kuroda Y, Wakao S, Kitada M, Murakami T, Nojima M, Dezawa M. Isolation, culture and evaluation of multilineage-differentiating stress-enduring (Muse) cells. Nature protocols. 2013;8:1391-1415.) and WO 2011/007900 (Japanese Patent No. 5185443), Dulbecco's Modified Eagle's Medium low-glucose (DMEM; Life Technologies, Carlsbad, CA, USA), 10% fetal bovine serum (FBS) (Hyclone; Thermo-Fisher Scientific, Waltham MA, USA), and 0.1 mg/mL kanamycin (Life Technologies) were used to culture the cells in a 10 cm dish at 37°C and 5% CO₂. Muse cells were separated from MSCs using 7 to 8 passages as follows. A rat anti-stage-specific embryonic antigen-3 (SSEA-3) IgM antibody (1:1000; BioLegend, San Diego, CA, USA) as a primary antibody and a goat anti-rat FITC µ-chain IgM antibody (1:1000; Miltenyi Biotec, Bergisch Gladbach, Germany) as an isotype control were reacted. A fluorescein isothiocyanate (FITC)-labeled anti-rat IgM antibody (1:100; Jackson ImmunoResearch Laboratories, Inc., West Grove, PA, USA) as a secondary antibody and anti-FITC micro beads (1:50; Miltenyi) as a tertiary antibody were reacted, and SSEA-3 positive cells were separated using autoMACS (registered trademark) Pro Separator (Miltenyi). After cell separation, the percentage of SSEA-3 positive cells in the cells enriched by magnetic cell sorting (MACS) was analyzed using BD FACS Aria (BD Biosciences, Franklin Lakes, USA) (FACS; fluorescence activated cell sorting). Cells containing more than 70% SSEA-3 positive cells were defined as Muse cells and used as transplantation cells.

Some MSCs were transduced with green fluorescent protein (GFP) or Nano-lantern/pcDNA3 using lentivirus with reference to the prior document (Hayase M, Kitada M, Wakao S, et al. Committed neural progenitor cells derived from genetically modified bone marrow stromal cells ameliorate deficits in a rat model of stroke. Journal of cerebral blood flow and metabolism: official journal of the International Society of Cerebral Blood Flow and Metabolism. 2009;29:1409-1420.), and reacted with a rat anti-SSEA-3 IgM antibody (1:1000; BioLegend) as a primary antibody and allophycocyanin (APC)-labeled anti-rat IgM antibody (1:100; Jackson ImmunoResearch) as a secondary antibody, and SSEA-3 positive cells and SSEA-3 negative cells were separated using FACS. As in the case of Muse cells separated by MACS, the separated cells were mixed in such a manner that the percentage of SSEA-3 positive cells was 70%.

### < Examination of Cell Transplantation Timing and Number of Transplanted Cells >

Cardiac function was evaluated by magnetic resonance imaging (MRI)(1T ICON, Bruker, Billerica, MA, USA) at 2, 3, 4, 6, and 8 weeks after myocarditis induction in order to determine the timing of cell transplantation as a preliminary experiment, and changes in cardiac function were compared.

In order to determine the optimal number of transplanted cells, three groups were prepared: 100,000 Muse cells, 200,000 Muse cells, and 400,000 Muse cells (n = 5 per group). Cell transplantation was performed at an optimal time for cell transplantation, and cardiac function was compared by MRI.

### < Cell Transplantation by Intravenous Administration >

Three weeks after myocarditis induction, the animals were randomly grouped as follows, and cell transplantation was performed: myocarditis model with saline (NS; normal saline) vehicle group (Vehicle group), MSC 200,000 cells group (MSC group), Muse 200,000 cells group (Muse group), and sham group without myocarditis induction (S group). For the purpose of examining the therapeutic effect of multiple doses, the group (Muse' group) in which 200,000 Muse cells were administered three weeks and four weeks after myocarditis creation, respectively, was prepared, and for the purpose of examining the therapeutic effect of MSCs containing SSEA-3 positive cells equivalent to those in the Muse cell group, a group (MSC' group) in which 3,000,000 MSCs were administered was prepared. Both types of cells for transplantation were suspended in 1 ml saline and administered transvenously. For cardiac function measurement and histological fibrosis evaluation experiments, cells enriched by MACS were administered. GFP-Muse cells separated by FACS were transplanted for immunohistochemistry experiments, and nano-lantern-Muse cells were transplanted for evaluation experiments of in vivo distribution. No immunosuppressive drugs were administered in either cell-treated group.

### < Measurement of Cardiac Function in Cell Transplanted Rats >

MRI (IT ICON, Bruker, Billerica, MA, US) imaging was performed under 1.5% isoflurane inhalation anesthesia in Week 2 and Week 8. Left ventricular short-axis images from the cardiac apex to the left ventricular outflow tract were performed with the following imaging parameters: repetition time 17 ms, echo time 1.72 ms, flip angle 30°, field of view 40×40 mm, slice thickness 1.25 mm, averages 8. The obtained imaging data were analyzed for the following items: left ventricular end-diastolic volume (EDV), left ventricular end-systolic volume (ESV), and ejection fraction (EF). Week 2 was evaluated by n = 9 per group, and Week 8 was evaluated by n = 6 per group.

Brain natriuretic peptide (BNP) in plasma of Week 2 was measured using enzyme-linked immunosolvent assay (ELISA) (BNP-32 Rat RIA Kit, Peninsula Laboratories, San Carlos, CA, USA).

### < Cardiac Histological Evaluation of Cell Transplanted Rats >

Rats were sacrificed to death by isoflurane overdose on Day 3, Week 2, and Week 8, and then perfused with 4% paraformaldehyde (PFA) and fixed. The heart was removed, divided into four 3-mm-thick sections in the short axis of the left ventricle, and embedded in paraffin or frozen tissue embedding material (O.C.T. Compound; Sakura Finetek, Tokyo, Japan). Thin sections were then prepared at 3 µm for paraffin sections and 6 µm for frozen sections. Sections at the level of the papillary muscle were used for the following histological evaluations.

For the purpose of confirming the degree of inflammation in heart tissue, hematoxylin-eosin (HE) staining was performed on paraffin sections. Slide glasses were examined under an optical microscope (BX53; Olympus, Tokyo, Japan).

In order to evaluate the differentiation potential of transplantation cells, fluorescence immunohistochemistry was performed in Week 2 and Week 8. A rabbit anti-GFP antibody (1;500; Medical & Biological Laboratories Co., Ltd., Nagoya, Japan), a goat Anti-ANP antibody (1: 100; Santa Cruz Biotechnology, Dallas, TX, USA), a goat anti-CD31 antibody (Santa Cruz Biotechnology), a mouse anti-sarcomeric alpha-actinin antibody (1:100; Sigma-Aldrich), a mouse anti-troponin-I antibody (1:50; Merck, Billerica, MA, USA), a mouse anti-connexin 43 antibody (1:50; Abcam, Cambridge, UK), and a mouse anti-alpha-SMA antibody (1:500; Thermo Fisher Scientific, Waltham, MA, USA) were used as primary antibodies, and an Alexa 488-labeled donkey anti-rabbit antibody (1:500; Jackson ImmunoResearch), an Alexa 594-labeled donkey anti-goat antibody (1:500; Jackson ImmunoResearch), and an Alexa 594-labeled donkey anti-mouse antibody (1:500; Jackson ImmunoResearch) were used as secondary antibodies, and the results were observed with a Nikon A1 confocal laser microscope (Nikon, Tokyo, Japan). ANP/GFP double positive cells/GFP positive cells and troponin-I/GFP double positive cells/GFP positive cells were calculated (n = 3).

In order to evaluate apoptosis in impaired tissues, TdT-mediated dUTP nick end labeling (TUNEL) staining was performed on Day 3 (In Situ Cell Death Detection Kit, TMR Red; Roche Diagnostics Deutschland GmbH, Mannheim, Germany). Twenty randomly selected 1 mm2 regions of interest (ROI) were observed, and the number of TUNEL-positive cells per unit area was calculated (n = 3 per group).

Masson-Trichrome staining was performed at Week 8 for evaluation of fibrosis. Slide glasses were observed and photographed with an optical microscope (Olympus). The cardiac fibrotic area/cardiac cross-sectional area was calculated using Adobe Photoshop (Adobe Inc., San Jose, CA, USA) (n = 6 per group).

### < Evaluation of Migration Factors in Impaired Tissues of Cell-transplanted Rats >

Measurement of sphingosine-1-phosphate (S1P), a migratory factor that induces Muse cells to the impaired site, was performed (Yamada Y, Wakao S, Kushida Y, et al. S1P-S1PR2 Axis Mediates Homing of Muse Cells Into Damaged Heart for Long-Lasting Tissue Repair and Functional Recovery After Acute Myocardial Infarction. Circulation research. 2018;122:1069-1083.). On Day 0, rats were sacrificed to death by isoflurane overdose, the heart was removed, and the left ventricle was homogenized by biomasher. A homogenate buffer (500 mM Tris-HCl/150 mM NaCl/ 1 mM EDTA/ 1% Triton X-100, 0.5% sodium deocyl sulfate/ 0.5% sodium deoxycholate) with protease inhibitor (cOmplete, Merck) and phosphatase inhibitor (PhosSTOP (trademark), Sigma-Aldrich) according to the product protocol was used. After centrifugation at 4°C for 15,000 rpm for 15 min, the concentration of SIP in the supernatant was measured by liquid chromatography-mass spectrometry (API 4000, AB/MDS SCIEX, Framingham, MA, USA) at Toray Research Center (Kamakura, Japan). The SIP concentration was expressed as the concentration in 1 g of heart tissue. The measurement was performed in the myocarditis non-induced group (Control group; n = 6) and in the myocarditis induced group (Myocarditis group; n = 6).

### < Evaluation of In Vivo Distribution of Transplanted Cells >

For the purpose of evaluating the in vivo distribution of transplanted cells, cells transduced with Nano-lantern were used (Saito K, Chang YF, Horikawa K, et al. Luminescent proteins for high-speed single-cell and whole-body imaging. Nature communications. 2012;3:1262.). Nano-lantern-Muse cell-treated rats, nano-lantern-MSC-treated rats, and non-treated rats (n = 3 per group) were treated with 200 µg/1 ml NS of selenoterazine by radial tail vein under sedation with isoflurane inhalation at Week 2. Rats were sacrificed to death by isoflurane overdose and the heart, lungs, brain, liver, pancreas, spleen, kidneys, stomach, small intestine, large intestine, femur, tibia, fibula, and quadriceps were removed. The heart was sliced into 3 mm thick slices and other organs into less than 1 cm thick slices and immersed in a 50 µg/ml solution of selenoterazine. Images were taken with IVIS Lumina LT (Perkin Elmer, Waltham, MA, USA), and the light intensity was quantified with Living Image Software (Perkin Elmer).

### < Evaluation of Angiogenesis >

At Week 2, 2 mg of dextran, Texas Red (Thermo-Fisher Scientific) was administered by radial tail vein under sedation with isoflurane inhalation. The rats were euthanized by isoflurane overdose and the hearts were removed. The tissues were made transparent according to Tissue transparency technique CUBIC (clear, unobstructed brain/body imaging cocktails and computational analysis) (Susaki EA, Tainaka K, Perrin D, et al. Whole-brain imaging with single-cell resolution using chemical cocktails and computational analysis. Cell. 2014;157:726-739.). After sufficient transparency was obtained, the cells were observed with a Multiphoton laser microscope (Nikon). One hundred and eighteen axial section still images of 0.78 × 0.78 µm were taken every 0.85 µm from the epicardial side to the intimal side. In the anterior, lateral, and posterior walls of the left ventricle of each sample, a total of nine images were taken, one at a time, randomly from the cardiac base, cardiac apex, and the middle of each wall, and the vascular lumen volume was calculated using Image J software (National Institutes of Health, Bethesda, MD, USA). Each group was evaluated at n = 3.

### < Statistical Analysis >

The obtained data were expressed as mean ± standard deviation. Statistical analysis was performed using JMP Pro 14 software (SAS Institute, Cary, NC, USA). Comparison of continuous variables between the two groups in the SIP value in tissue and signal intensity of IVIS data proved to be normally distributed by Shapiro-Wilk test, and Student's t-test was performed. One-way ANOVA and Tukey-Kramer's post hoc test were used for other multiple group comparisons. Significance level was set at p < 0.05.

### << Experimental Results >>

### < SSEA-3-positivity in Cell Population After Enrichment of Muse Cells >

The flow cytometry gate was set to prevent the detection of SSEA-3 positive cells in MSCs reacted with isotype control. The SSEA-3 positivity of MSCs used in this study was 5.1±1.3%, and the SSEA-3 positivity after cell separation by MACS was 75.0±4.8% (FIG. 1).

### < Examination of Optimal Cell Transplantation Timing and Number of Transplanted Cells >

In order to examine the optimal timing of cell administration, the degree of inflammation at 2, 3, 4, 6, and 8 weeks after induction of myocarditis was evaluated histologically, and the changes in EF were compared using MRI. HE staining showed mononuclear cell infiltration at Week 2, but no other inflammatory findings. At Week 3, there was marked granulocyte and mononuclear cell infiltration, accompanied by cardiomyocyte degeneration and interstitial edema (A of FIG. 2). At Week 6, the granulocyte and mononuclear cell infiltrates had decreased, and MT staining showed the appearance of fibrosis. Both lesions were almost circumferential on the epicardial side. The EF decreased over time up to Week 3, and plateaued after Week 3 (B in FIG. 2). In this study, in order to achieve a clinically relevant validation, it was decided to perform cell transplantation three weeks after myocarditis induction, when cardiac function and histological severity peaked.

Then, the optimal number of transplanted cells was examined. Three weeks after induction of myocarditis, the 100,000 Muse cell group, 200,000 Muse cell group, and 400,000 Muse cell group were prepared, and the EF was measured two weeks after cell transplantation (FIG. 3). The EF was 74.0±2.5% in the 200,000 Muse cell group, which was statistically significantly higher than that in the 100,000 Muse cell group (69.5±2.4%, p<0.05), but not statistically significantly different from that in the 400,000 Muse cell group (72.7±1.3%). Based on the above, the number of transplanted cells was set at 200,000 cells.

### < Muse Cell Migration Factors and In Vivo Distribution >

The concentration of SIP, a migration factor of Muse cells, was measured using cardiac tissue homogenate samples on Day 0 (FIG. 4). The concentration of SIP was 24.9±2.9 ng/g in the control group and 32.5±4.9 ng/g in the myocarditis group, and was statistically significantly higher in the myocarditis group (p < 0.05).

The in vivo distribution of the administered cells at Week 2 was evaluated by IVIS (A in FIG. 5). The light intensity of each organ obtained by IVIS is illustrated (B in FIG. 5). More cells were accumulated in the Muse group than in the MSC group (p < 0.05). The cells were mainly accumulated around the epicardial side, which was consistent with the inflammation site caused by this disease model. The MSC group showed a slight accumulation in the lungs, while the Muse group showed no accumulation in the lungs. No accumulation in other organs was observed in either group.

### < Improvement Effect on Cardiac Function in Cell Transplanted Rats >

Cardiac function at Week 2 and Week 8 is shown (A-F in FIG. 6). At Week 2, the EF in the Muse group was 73.1±1.9%, which was statistically significantly higher than that in the Vehicle group (60.8±2.5%, p < 0.001), MSC group (66.7±2.4%, p < 0.001), and MSC' group (68.3±2.3%, p < 0.01). There was no significant difference between the Muse group and the Muse' group (73.2±2.0%). ESV in Muse group was 0.11±0.01 ml, which was statistically significantly lower than that in Vehicle group (0.15±0.01 ml, p < 0.001). There was no significant difference between the Muse group and the MSC group (0.12±0.02 ml) and MSC' group (0.12±0.01 ml), but there was a trend toward lower values in the Muse group. EDV was not significantly different between the cell transplantation groups.

At Week 8, the EF in the Muse group was 68.8±4.5%, which was statistically significantly higher than that in the Vehicle group (59.5±3.9%, p < 0.001). There was no significant difference between the Muse group and the Muse' group (70.1±3.4%). There was no significant difference in EDV and ESV between the cell transplanted groups.

The BNP levels in plasma at Week 2 are illustrated (G in FIG. 6). The BNP level in the Muse group was 347.2±188.6 pg/ml, which was statistically significantly lower than that in the Vehicle group (980±241.1 pg/ml, p < 0.001), MSC group (802.2±171.1 pg/ml, p < 0.01), and the MSC' group (770±316.3 pg/ml, p < 0.01). There was no statistically significant difference between Muse group and Muse' group (395.2±283.3 pg/ml).

### < Histological Evaluation of Cell Transplanted Rats >

The differentiation potential of transplanted GFP-Muse cells into cardiomyocytes and vascular component cells at Week 2 was observed histologically. Among GFP-positive cells, cells expressing ANP (immature myocardial marker), sarcomeric α-actinin, troponin-I, and connexin 43 (mature myocardial markers), and αSMA, and CD31 which are vascular component cells were observed (A-F in FIG. 7). The percentage of ANP⁺GFP⁺ double positive cells among GFP positive cells was 41.3 ± 1.8%, and the percentage of troponin-I⁺GFP⁺ double positive cells was 16.2 ± 3.1%.

For the purpose of evaluating the anti-fibrotic/fibrotic effect of transplanted cells, cardiac tissue sections from Week 8 were observed by Masson-Trichrome staining. A representative image of each group is illustrated in A in FIG. 8. The percent fibrotic area of each group was calculated as cardiac fibrotic area/cardiac cross-sectional area × 100 (B in FIG. 8). The percent fibrotic area of the Muse group (6.0±2.0%) and the Muse' group (4.8±3.4%) were all statistically significantly lower than that of the Vehicle group (12.1±4.5%). There was no significant difference between the Muse group and the MSC group (8.6±2.6%) and the MSC' group (8.5±4.5%), but there was a trend toward lower values in the Muse group.

### < Comparison of Apoptotic Cell Numbers in Cell Transplanted Rat Tissues >

In order to evaluate the anti-apoptotic effect of cell transplantation, the numbers of TUNEL-positive cells between the groups were compared using cardiac tissue samples on Day 3. A representative image of each group is illustrated in FIG. 9. The number of TUNEL-positive cells in the Muse group was 1.8±0.4 cells/mm², which was statistically significantly lower (p = 0.001) than that in the Vehicle group (7±1.6 cells/mm²) (B of FIG. 9).

### < Evaluation of Angiogenesis by Muse Cells >

For the purpose of evaluating angiogenesis by cell transplantation, the volume of blood vessels in the transparent heart tissue was measured. The tomographic images of each group taken by multiphoton microscopy are illustrated in A in FIG. 10. The percentage of vascular volume in cardiac tissue was 7.4±1.2% in the Muse group, which was statistically significantly higher (p = 0.047) than that in the Vehicle group (3.2±0.5%) (B in FIG. 10).

As described above, the efficacy of Muse cells for the treatment of myocarditis was demonstrated.

### Industrial Availability

When administered to a patient who has developed myocarditis, the cell product of the present invention can accumulate in the impaired area of myocarditis, repair the impaired site (proliferation of cardiomyocytes, angiogenesis, tissue repair, and the like), and improve or restore cardiac function, and can be applied to the treatment of myocarditis.

## Claims

1. A cell product for treating myocarditis, comprising a SSEA-3-positive pluripotent stem cell derived from a mesenchymal tissue in a living body or a SSEA-3-positive pluripotent stem cell derived from a cultured mesenchymal cell.

2. The cell product of claim 1, wherein the pluripotent stem cell contains a concentrated cell fraction.

3. The cell product of claim 1 or 2, wherein the pluripotent stem cell is CD105-positive.

4. The cell product of any one of claims 1 to 3, wherein the pluripotent stem cell is CD117-negative and CD146-negative.

5. The cell product of any one of claims 1 to 4, wherein the pluripotent stem cell is CD117-negative, CD146-negative, NG2-negative, CD34-negative, vWF-negative, and CD271-negative.

6. The cell product of any one of claims 1 to 5, wherein the pluripotent stem cell is CD34-negative, CD117-negative, CD146-negative, CD271-negative, NG2-negative, vWF-negative, Sox10-negative, Snail-negative, Slug-negative, Tyrp1-negative, and Dct-negative.

7. The cell product of any one of claims 1 to 6, wherein said pluripotent stem cell is one having all of the following characteristics:
(i) having low or no telomerase activity;
(ii) capable of differentiating into any of tridermic cells;
(iii) showing no neoplastic proliferation; and
(iv) having self-renewal capacities.

8. The cell product of claim 7, wherein said pluripotent stem cell is one having all of the following characteristics:
(i) SSEA-3-positive;
(ii) CD105-positive;
(iii) having low or no telomerase activity;
(iv) capable of differentiating into any of tridermic cells;
(v) showing no neoplastic proliferation; and
(vi) having self-renewal capacities.

9. The cell product of any one of claims 1 to 8, wherein the myocarditis is one or two or more selected from the group consisting of lymphocytic myocarditis, giant cell myocarditis, eosinophilic myocarditis, and granulomatous myocarditis.
